# EUROPEAN PATENT APPLICATION

(11) **EP 4 560 014 A1**
(43) Date of publication of application: **28.05.2025**
(21) Application number: 23843425.2
(22) Date of filing: 21.07.2023
(51) Int. Cl.: C12N 7/00, A61K 39/215, A61P 31/14

(54) **ATTENUATED STRAIN OF AVIAN INFECTIOUS BRONCHITIS VIRUS AND VACCINE COMPOSITION CONTAINING SAME**

(30) Priority: 22.07.2022 KR 20220091378; 13.09.2022 KR 20220115199
(71) Applicant: Khav Co., Ltd., Anseong-si, Gyeonggi-do 17608 (KR)
(72) Inventor: YOUN, Ha Na, Hanam-si, Gyeonggi-do 12980 (KR); LEE, Hyukchae, Seoul 05099 (KR)
(74) Representative: J A Kemp LLP
(86) International application number: PCT/KR2023/010551
(87) International publication number: WO 2024/019579

(57) **Abstract**

The present invention relates to an attenuated strain of avian infectious bronchitis virus and a vaccine composition containing same. With weak pathogenicity and high immunogenicity, the attenuated strain of the present invention can induce defensive capabilities against the avian infectious bronchitis virus and thus can be advantageously used as an attenuated live vaccine.

## Description

### Technical Field

The present invention was made with the support of the Ministry of SMEs and Startups of the Republic of Korea under Project No. S3037458, which was executed in the research project named "Development of Globally Applicable Vaccine for Chicken Infectious Bronchitis Virus" in the research program titled "SME Technology Innovation Development Program (Market Expansion Type)" by KHAV CO., LTD under the management of the Korea Technology and Information Promotion Agency for SMEs, from 18 December 2020 to 17 December 2022.

This application claims priority and the benefit of Korean Patent Application No. 10-2022-0091378 filed in the Korean Intellectual Property Office on 22 July 2022, the disclosure of which is incorporated herein by reference.

This application claims priority and the benefit of Korean Patent Application No. 10-2022-0115199 filed in the Korean Intellectual Property Office on 13 September 2022, the disclosure of which is incorporated herein by reference.

The present invention relates to an attenuated strain of avian infectious bronchitis virus and a vaccine composition containing the same. More specifically, the present invention relates to an avian infectious bronchitis virus (IBV) QX 1830029 HP70 strain, obtained by attenuating the avian IBV/Korea/289/2018 strain through serial subculture at a temperature as high as 60°C-70°C, and to an avian infectious bronchitis virus vaccine composition using the same.

### Background Art

Avian infectious bronchitis (hereinafter, IB) is an acute respiratory infectious disease in chickens caused by avian infectious bronchitis virus (hereinafter, IBV). IB has a high morbidity of nearly 100% and causes an overall productivity reduction, such as coughing, runny nose, a decreased rate of weight gain, and reduced egg production accompanied by a decline in both the external and internal egg quality. The death rate of chickens caused by IBV infection itself is not high, but most IB cases are accompanied by secondary bacterial infections, such as colibacillosis, which results in a high death rate and additional economic loss, such as antibiotic administration costs. This has caused enormous economic damage in most countries involved in poultry farming.

IBV, belonging to the genus Gammacoronavirus of the family Coronaviridae, is a pathogen causing this disease. From the initial diagnosis of IBV in the 1930s in the United States until the 1940s, IBV was known to exist only in a Massachusetts (hereinafter, Mass) serotype, called the respiratory type, but after the identification of a new serotype distinct from the respiratory type, numerous other serotypes have been continuously reported worldwide. Compared with other viruses, IBV has a very high mutation rate in the Spike gene, which encodes a main antigenic determinant protein, and exhibits genetic mutations arising through various mechanisms, such as recombination and self-mutation within the viral gene, and thus more than a dozen serotypes have been reported, with new serotypes still emerging.

Regarding IB outbreaks in Korea, since the first respiratory-type IBV (hereinafter, GI-15 IBV) was isolated and reported in 1986, IBV infection has caused respiratory symptoms and persistently disrupted egg laying accompanied by deformed eggs. Since the Korean isolate nephropathogenic IBV (hereinafter, KM91-like IBV) was reported in the 1990s, the nephropathogenic IBV began to account for the majority of IB cases occurring in Korea. From the early 2000s, the nephropathogenic IBV (hereinafter, QX-like subgroup I IBV) originating from China has also occurred, and then the IBV epidemic strains have been reported to exhibit persistent mutation, such as the emergence of viruses formed through the recombination of two types. Since the cross-protective capacity between different serotypes deteriorates due to the nature of IBV, current vaccines in the poultry industry are being developed to match the serotypes of IBV circulating in each country. The Massachusetts serotype (Mass type) preventive vaccines (e.g., H120 strain) developed in the late 1980s, immediately after the first identification of IB outbreaks, are still used in the forms of live and killed vaccines, and since 1997 when KM91-like IBV was identified, killed vaccines using the KM91 strain have been developed, and used together with the Mass type vaccines. Subsequently, K-II live and killed vaccines using K2 strain, which has the same genotype as the KM91 strain, were developed. However, despite the use of various vaccine strains, the damage caused by IB in the poultry farms is still ongoing due to a difference in protective type of existing vaccines against pandemic strains, quality issues of vaccines, or the like.

Especially, the nephropathogenic IBV (hereinafter, IBV QX-like subgroup III IBV) that has been recently prevalent to cause damage in Korea was confirmed to have relatively low genetic homology with viruses that were prevalent in the past, such as forming a cluster that is genetically and statistically different from the isolates belonging to KM91-like IBV that were prevalent in the 1990s and the isolates belonging to QX-like subgroup I IBV that was originated from China in the early 2000s (Genomic Analysis of Avian Infectious Bronchitis Viruses Recently Isolated in South Korea Reveals Multiple Introductions of GI-19 Lineage (IBV QX Genotype), Viruses (2021 May) 31;13(6), 1045). However, there are no vaccines developed against IBV belonging to the latest epidemic QX-like subgroup III.

Throughout the specification, many papers and patent documents are referenced and their citations are provided. The disclosures of cited papers and patent documents are entirely incorporated by reference herein, and the level of the technical field within which the present invention falls and details of the present invention are explained more clearly.

### Disclosure of Invention

### Technical Problem

The present inventors have made intensive research efforts to develop and manufacture a preventive vaccine based on recently circulating IBV. As a result, the present inventors produced the IBV QX 1830029 HP70 strain obtained by attenuating the IBV/Korea/289/2018 virus, belonging to the QX-like subgroup III genotype of IBVs recently circulating in Korea and established a preventive effect thereof against avian infectious bronchitis infection, and thus completed the present invention.

Accordingly, an aspect of the present invention is to provide an attenuated avian infectious bronchitis virus QX 1830029 HP70 strain.

Another aspect of the present invention is to provide a vaccine composition for preventing an avian infectious bronchitis virus, the vaccine composition containing the attenuated avian infectious bronchitis virus QX 1830029 HP70 strain.

Other purposes and advantages of the present disclosure will become more obvious when taken with the following detailed description of the invention, claims, and drawings.

### Solution to Problem

In accordance with an aspect of the present invention, there is provided an avian infectious bronchitis virus QX 1830029 HP70 strain.

In an embodiment of the present invention, the QX 1830029 HP70 strain is attenuated from avian infectious bronchitis virus IBV/Korea/289/2018.

The QX 1830029 HP70 strain according to an embodiment of the present invention has been deposited on 11 August 2022 with the Korean collection for type cultures (KCTC) at Korea Research Institute of Bioscience and Biotechnology, and assigned accession number KCTC 15049BP.

In an embodiment of the present invention, a spike protein of the QX 1830029 HP70 strain includes the amino acid sequence of SEQ ID NO: 6.

In an embodiment of the present invention, a spike protein of the QX 1830029 HP70 strain is encoded by the nucleotide sequence of SEQ ID NO: 4.

In an embodiment of the present invention, the avian infectious bronchitis virus QX 1830029 HP70 strain is obtained by attenuating IBV/Korea/289/2018 virus through sub-culturing.

In an embodiment of the present invention, the sub-culturing is conducted by heat treatment at a temperature of 60 to 70°C.

In an embodiment of the present invention, the sub-culturing is conducted in embryonated chicken eggs.

In an embodiment of the present invention, the sub-culturing is repeated for 50 to 70 passages.

In an embodiment of the present invention, the heat treatment is conducted for 20 minutes to 3 hours or 20 minutes to 2 hours.

In accordance with another aspect of the present invention, there is provided a vaccine composition for preventing an avian infectious bronchitis virus, the vaccine composition containing the foregoing avian infectious bronchitis virus QX 1830029 HP70 strain.

In an embodiment of the present invention, the vaccine is an attenuated vaccine or an inactivated vaccine.

The vaccine according to the present invention may be manufactured by a conventional method, such as one that is commonly employed for commercially available IBV attenuated vaccines and IBV inactivated vaccines. Briefly, cells or individuals susceptible to IBV are inoculated with the IBV according to the present invention and then propagated until the virus replicates to a desired infectious titer, and thereafter, an IBV-containing substance is harvested, optionally attenuated or inactivated, and then mixed with a pharmaceutically acceptable carrier or diluent.

The attenuated vaccine according to the present invention containing the live virus of the present invention may be manufactured and marketed in a suspension form or a lyophilized form, and further contains a pharmaceutically acceptable carrier or diluent that is commonly used in such a composition. Examples of the carrier include a stabilizer, a preservative, and a buffer. A suitable example of the stabilizer is SPGA, a carbohydrate (e.g., sorbitol, mannitol, starch, sucrose, dextran, glutamate, or glucose), a protein (e.g., albumin or casein), or a degradation product thereof. A suitable example of the buffer is an alkali metal phosphate. Suitable examples of the preservative include thimerosal, merthiolate, and gentamicin. Examples of the diluent includes water, an aqueous buffer (e.g., PBS), an alcohol, and a polyol (e.g., glycerol).

If needed, the attenuated live vaccine according to the present invention may contain an immune adjuvant. Examples of suitable compounds and compositions having immunoadjuvant activity are as stated below.

Although administration by injection, for example, intramuscular injection, subcutaneous injection, or in ovo, of the live vaccine according to the present invention is possible, the vaccine is preferably administered by an inexpensive mass application route commonly used for IBV vaccination. For IBV vaccination, this route includes drinking water, spray, and aerosol vaccinations.

Alternatively, the present invention provides a vaccine containing IBV in an inactivated (killed) form. The advantage of inactivated IBV vaccines is that high levels of protective antibodies can be maintained for a long period.

The purpose of inactivating the viruses harvested after the propagation step is to eliminate reproduction of the viruses. This can be generally achieved by chemical or physical means well known in the art.

A vaccine containing inactivated IBV may, for example, comprise at least one of the foregoing pharmaceutically acceptable carriers or diluents suited for this purpose.

Preferably, the inactivated vaccine according to the invention comprises at least one compound with immunoadjuvant activity. Suitable compounds or compositions for this purpose include aluminum hydroxide, aluminum phosphate, or aluminum oxide, for example, oil-in-water or water-in-oil emulsions (e.g., Bayol F^{®} or Marcol 52^{®}) based on mineral oils, or vegetable oils such as vitamin E acetate and saponins.

The vaccine according to the present invention comprises an effective dosage of the isolated IBV of the present invention as an active ingredient, i.e., an amount of IBV that induces immunity in vaccinated birds against challenge by wild IBV. As used herein, the term immunity is defined as the induction of a significantly higher level of protective effect in a population of vaccinated birds, compared with an unvaccinated group.

In general, the live vaccine according to the invention may be administered in a dose of 100-109 TCID50 per animal, preferably 103-106 TCID50 per animal. Inactivated vaccines may contain an antigenic equivalent of 106-1010 TCID50 per animal.

Inactivated vaccines are usually administered parenterally, for example, intramuscularly or subcutaneously.

The IBV vaccine according to the present invention may be effectively used in chickens, and may also be used in other poultry, such as turkeys and guinea fowl. Examples of chickens include pullets, broilers, and laying hens.

The age of the animals receiving the attenuated live vaccine or inactivated vaccine according to the invention is the same as that of the animals receiving a conventional IBV-attenuated lives vaccine or IBV-inactivated vaccines. For example, pullets (free of maternally derived antibodies-MDA) may be vaccinated at 1 day of age or in ovo, whereas pullets with high levels of MDA are preferably vaccinated at 2-3 weeks of age. Laying hens or broilers with low levels of MDA may be vaccinated at 1 -10 days of age, followed by booster vaccinations with inactivated vaccine at 6-12 and 16-20 weeks of age.

The present invention also includes combinative vaccines comprising, in addition to the above-described IBV, other pathogens that are infectious to poultry or one or more vaccine components prepared therefrom.

In a specific embodiment of the present invention, the combinative vaccine further comprises at least one pathogen, such as Mareks disease virus (MDV), infectious bursal disease virus (IBDV), Newcastle disease virus (NDV), egg drop syndrome (EDS) virus, turkey rhinotracheitis virus (TRTV), or Reovirus, or a vaccine manufactured therefrom.

In accordance with another aspect of the present invention, there is provided a method for preventing an avian infectious bronchitis virus, the method including administering the vaccine composition for preventing the avian infectious bronchitis virus to poultry in need of vaccination.

In an embodiment of the present invention, the administration may be conducted through eye drop inoculation, spray inoculation, drinking water inoculation, or a combination thereof.

In an embodiment of the present invention, the administration may be conducted at least one time.

In an embodiment of the present invention, the administration may be conducted at a time corresponding to 1 day of age, 6-8 weeks of age, 12-14 weeks of age, or a combination thereof.

In a specific embodiment of the present invention, the administration at 1 day of age may be conducted by eye drop inoculation or spray inoculation.

In a specific embodiment of the present invention, the administration at 6-8 weeks of age may be conducted by drinking water inoculation.

In a specific embodiment of the present invention, the administration at 12-14 weeks of age may be conducted by intramuscular or subcutaneous injection of a killed vaccine, but is not limited thereto. The killed vaccine may be a killed oil vaccine.

Additionally, the killed vaccine may be a killed vaccine obtained by inactivating the infectious bronchitis virus QX 1830029 HP70 strain of the present invention, or a conventional killed vaccine using another virus vaccine strain.

### Advantageous Effects of Invention

The present invention relates to an attenuated strain of avian infectious bronchitis virus and a vaccine composition containing the same. The attenuated avian infectious bronchitis virus QX 1830029 HP70 strain of the present invention has low pathogenicity and excellent immunogenicity, thereby inducing the protective capacity against avian infectious bronchitis virus, and thus can be advantageously used as an attenuated live vaccine. Considering that QX-type IBV poses a threat in Korea as well as most countries including China, Japan, Southeast Asia, and Europe, the present invention can contribute to preventing the productivity decline in the global poultry industry.

### Brief Description of Drawings

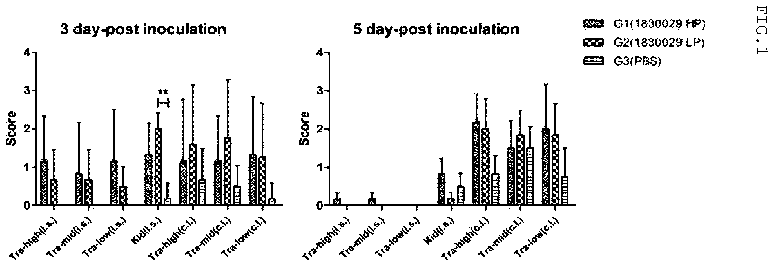

FIG. 1 shows the results of tissue safety evaluation of 3 day- and 5 day-post eye drop inoculation of IBV QX 1830029 low passage and HP70 strains in 1-day-old SPF chicks (Two-way ANOVA, Bonferroni posttests between each column).
FIG. 2 shows the results of tissue safety evaluation of 7 day- and 10 day-post eye drop inoculation of IBV QX 1830029 low passage and HP70 strains in 1-day-old SPF chicks (Two-way ANOVA, Bonferroni posttests between each column).

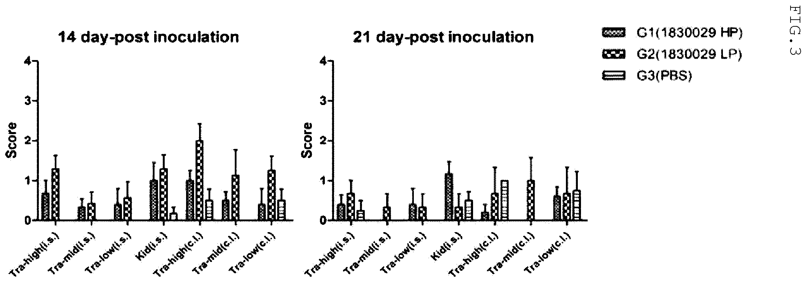

FIG. 3 shows the results of tissue safety evaluation of 14 day- and 21 day-post eye drop inoculation of IBV QX 1830029 low passage and HP70 strains in 1-day-old SPF chicks (Two-way ANOVA, Bonferroni posttests between each column) .

### Best Mode for Carrying out the Invention

Hereinafter, the present invention will be described in more detail with reference to exemplary embodiments. These exemplary embodiments are provided only for the purpose of specifically illustrating the present invention, and therefore, according to the purpose of the present invention, it would be apparent to a person skilled in the art that these exemplary embodiments are not construed to limit the scope of the present invention.

### Examples

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Example 1: Isolation and identification of infectious bronchitis virus (IBV) from outdoor farmers in Korea

In 2018, the trachea and kidney were taken from a chicken suspected of avian infectious bronchitis virus infection in a native chicken farm in Korea, and then mixed with sterile phosphate buffered saline (PBS, pH 7.2) to prepare 10% by weight of an emulsion. The prepared emulsion was centrifuged at 3,000 g for 15 minutes to precipitate cells and tissues, and the supernatant was filtered through a 0.45-µm syringe filter to extract live viruses. The extracted viruses were inoculated into the allantoic cavity (AC) of 11-day-old SPF embryonated eggs, and the viruses were collected after 48-72 hours of culture. After centrifugation at 2,000 g for 10 minutes in order to separate red blood cells incorporated during the collection, the presence of an avian infectious bronchitis virus in the supernatant was verified through reverse transcription polymerase chain reaction (RT-PCR).

### Example 2: Attenuation of virus through serial subculture and heat treatment in embryonated chicken eggs

To use isolated IBV/Korea/289/2018 virus (hereinafter, IBV QX 1830029 low passage) as a live vaccine strain through the weakening of pathogenicity, the corresponding strain was serially subcultured in 10-day-old SPF embryonated chicken eggs. The serial subculture in chicken embryos for attenuation was conducted repeatedly until the original pathogenicity of the virus was weakened, as described by Jackwood et al. (Rapid heattreatment attenuation of infectious bronchitis virus, Avian Pathology (June 2010) 39(3), 227-233). As a result of subculture for 70 passages, the IBV QX 1830029 Heat passage 70 (hereinafter, "IBV QX 1830029 HP70") strain was obtained.

Usual IBV isolates are generally heat-sensitive so that the isolates are inactivated by heat treatment at 56°C for 15 minutes, but the IBV QX 1830029 low passage strain were not easily attenuated due to its strong heat resistance, unlike other IBVs. Therefore, the present inventors conducted attenuation under temperature conditions changing from 60°C to 70°C during serial subculture. The temperature and time for heat treatment are shown in Table 1 below.

**[TABLE 1]**

| Subculture procedure and heat treatment temperature and time for securing IBV QX 18300029 HP70 strain | | |
|---|---|---|
| Number of passages | Heat treatment temperature | Heat treatment time |
| CE1-CE33 | 65°C | 120 min |
| CE34-CE41 | 70°C | 45 min |
| CE42-CE63 | 62°C | 25 min |
| CE64-CE70 | 60°C | 20 min |

### Example 3: IBV QX 1830029 HP70 strain sequencing and homology comparison with field isolates

To identify the change according to the attenuation of the avian infectious bronchitis virus, the present inventors conducted sequencing comparison of S1 protein, which determines neutralizing ability and serotype of the virus, including neutralization and serotype-specific epitopes.

The genetic homology comparison between the IBV QX 1830029 low passage strain and the HP70 strain was conducted by sequencing after the expression through a reverse transcription polymerase chain reaction (RT-PCR) using the forward primer nucleotide sequence (20,180-F: TGGAAAAACACTGCACGCAA) of SEQ ID NO: 1 and the reverse primer nucleotide sequence (24,216-R: TCACCTGAACAATCGTCAGCT) of SEQ ID NO: 2.

Table 2 and Table 3 show the spike protein gene sequencing results of IBV QX 1830029 low passage and HP70 strains, respectively.

Additionally, S1 protein sequencing and phylogenetic analysis of main IBV isolates were conducted to analyze the phylogenetic characteristics of the field strain IBV/Korea/289/2018 virus (IBV QX 1830029 low passage), and as a result, the IBV/Korea/289/2018 virus (IBV QX 1830029 low passage) was confirmed to belong to the IBV QX-like III subgroup that has been recently circulating in Korea.

**[TABLE 2]**

| Sequencing results of spike protein of IBV QX 1830029 low passage strain | | |
|---|---|---|
| Virus | Spike protein sequence | SEQ ID NO: |
| IBV QX 1830029 low passage | GTGGTTTATGGTTTAATTCTTTGTCAGTTTCTCTTACTTATG GACCCCTACAGGGAGGGTGTAAGCAATCTGTTTTTAGTGGTA AGGCAACGTGTTGTTATGCCTACTCTTATAATGGCCCTAGGG CATGTAAAGGTGTTTATTCAGGTGAATTAAGCAAGACTTTTG AATGTGGATTGCTGGTTTATGTTACTAAGAGTGATGGCTCTC GTATACAGACTAGAACAGAGCCCTTAGTATTAACGCAACACA ATTATAATAATATTACTTTAGATAAGTGTGTTGACTATAATA TATATGGCAGAGTAGGCCAAGGTTTTATTACTAATGTGACTG ATTCTGCTGCTAATTCTAGTTATTTAGCAGATGGTGGGTTAG CTATTTTAGATACTTCGGGTGCCATAGATGTCTTTGTTGTAC AGGGCAGCTATGGTCTTAATTATTACAAGGTTAATCCTTGTG AAGATGTTAATCAACAGTTTGTAGTGTCTGGTTTGGTGCTAT TTCATCAGTAATTCAAGACATTTATGCGCAACTTGATGCAAT TCAAGCAGATGCACAAGTAGACCGTCTTATTACTGGCAGACT CTCTTCTCTCTCAGTTCTAGCCTCTGCTAAGCAATCTGAGTA CATCAGAGTTTCCCAACAACGTGAATTGGCTACTCAAAAAAT TAATGAGTGTGTTAAATCACAATCTAATAGGTATGGTTTTTG TGGTAGTGGAAGACATGTTCTTTCGATACCACAAAATGCACC TAATGGTATAGTGTTTATACACTTTAGTTACACTCCAGAGAG TTTTGTTAATGTTACTGCAATAGTAGGTTTTTGTGTACAGCC TGCTAATGCTAGTCAGTATGCAATAGTGCCTGTTAATGGTAG AGGTATTTTTATACAAGTTAATGGTAGTTACTATATCACTGC ACGTGATATGTATATGCCTAGAGATATTACTGCAGGAGATGT AGTTACTCTTACTTCTTGTCAAGCAAATTATGTTAATGTAAA TAAAACCGTCATTACTACATTTGTGGAAGATGACGATTTTGA TTTTGATGATGAGTTGTCGAAATGGTGGAATGATACTAAGCA TGAGCTACCAGACTTTGACGACTTCAATTATACAGTACCCAT ACTTAATATTAGTGGTGAAATTGATCATATTCAAGGTGTTAT ACAGGGTCTTAATGACTCCCTTATAAACCTTGAAGAACTTTC AATACTTAAAACTTATATTAAGTGGCCTTGGTATGTTTGGCT AGCCATAGGCTTTGCTATTATTATCTTTATCCTTATTCTAGG ATGGGTGTTTTTCATGACAGGTTGTTGTGGTTGTTGTTGTGG ATGCTTTGGTATCATTCCTCTAATGAGTAAGTGTGGTAAGAA ATCATCTTATTACACGACTTTTGATAATGATGTGGTAACTGA ACAATACAGACCTAAAAAGTCTGTTTAA | 3 |

**[TABLE 3]**

| Sequencing results of spike protein of IBV QX 1830029 HP70 strain | | |
|---|---|---|
| Virus | Spike protein sequence | SEQ ID NO: |
| IBV QX 1 8 3 0 0 2 9 HP70 | ACTACATTTGTGGAAGATGACGATTTTGATTTTGATGATGAG TTGTCGAAATGGTCATGAGCTACCAGACTTTGACGACTTCAA TTATACAGTACCCATACTTAATATTAGTGGTGAAATTGATCA TATTCAAGGTGTTATACAGGGTCTTAATGACTCCCTTATAAA CCTTGAAGAACTTTCAATACTTAAAACTTATATTAAGTGGCC TTGGTATGTTTGGCTAGCCATAGGCTTTGCTATTATTATCTT TATCCTTATTCTAGGATGGGTGTTTTTCATGACAGGTTGTTG TGGTTGTTGTTGTGGATGCTTTGGTATCATTCCTCTAATGAG | 4 |
| | TAAGTGTGGTAAGAAATCATCTTATTACACGACTTTTGATAA TGATGTGGTAACTTAA | |

### Example 4: Evaluation on attenuation of IBV QX 1830029 HP70 strain

### 4-1. Evaluation on safety of IBV QX 1830029 HP70 strain in eye drop inoculation

To evaluate whether IBV QX 1830029 HP70 strain, the attenuated strain of the present invention, was successfully attenuated, a pathogenicity comparison test was conducted through eye drops of IBV QX 1830029 lowpassage and HP70 strains.

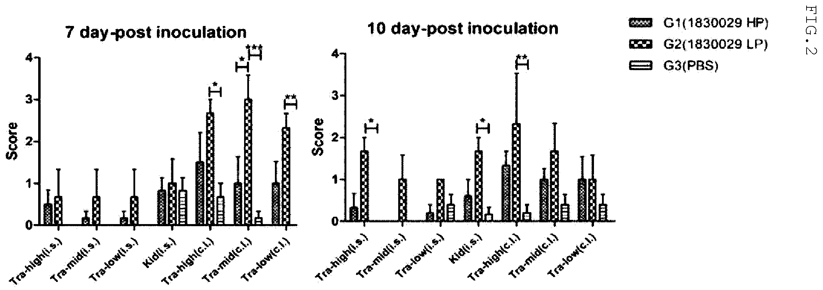

The negative control group was inoculated with the same amount of sterile phosphate buffered saline (PBS) and observed for 21 days. On days 3, 5, 7, 10, 14, and 21 after vaccination, appropriate amounts of samples were extracted from each of test and control groups, and evaluated for safety on the basis of the tissue lesion index and the ciliostasis index of the trachea and kidney. The results are shown in Table 4 and FIG. 2.

**[TABLE 4]**

| Comparison results of clinical symptoms and death rate for 21 days after eye drop inoculation of IBV QX 1830029 lowpassage and HP70 strains in 1-day-old SPF chicks | | |
|---|---|---|
| Group | Inoculation does | Death^{B} |
| G1: IBV QX 1830029 HP70 strain (1830029 HP) | 10^{4.5} EID₅₀/animal | 0/36 |
| G2: IBV QX 1830029 low passage strain (1830029 LP) | 10^{4.5} EID₅₀/animal | 7/35 |
| G3: Negative control group | - | 0/36 |

^{A} Number of clinical symptom expressed animals after eye drop inoculation / total number of tested animals

^{B} Number of dead animals after eye drop inoculation / total number of tested animals

As a result of evaluating the attenuation through eye drop inoculation, no clinical symptoms or death rate were observed in the IBV QX 1830029 HP70 strain and the negative control group, but a death rate of 20% for 21 days was observed in the IBV QX 1830029 low passage strain.

Additionally, as a result of histological lesion comparison, a statistically significant difference was observed in the IBV QX 1830029 low passage strain compared with the negative control group and the HP70 strain, but similar results were observed in the IBV QX 1830029 HP70 strain compared with the negative control group.

It was therefore identified that the IBV QX 1830029 HP70 strain, the attenuated strain of the present invention, showed weakened pathogenicity, indicating its safety for eye drop inoculation in 1-day-old SPF chicks.

### 4-2. Evaluation on safety of IBV QX 1830029 HP70 strain for spray inoculation

To evaluate the safety of IBV QX 1830029 HP70 strain, the attenuated strain of the present invention, in spray inoculation, a tissue safety comparison test was conducted through spray inoculation of IBV QX 1830029 HP70 strain and a control vaccine. The control vaccine was selected from vaccines that are commercially available and widely used in Korea.

A total of 60 1-day-old SPF chicks were divided into an IBV QX 1830029 HP70 group, a control vaccine group, and a negative control group, each with 20 animals, and then spray inoculated with 10^{4.5} EID₅₀/animal. The negative control group was inoculated with the same amount of sterilized phosphate buffered saline (PBS). At 5 day-post inoculation, 10 animals from each of the test and control groups were necropsied to collect the tracheas and kidneys, which were then fixed in 10% formalin, and thereafter, safety evaluation was conducted by determining the tissue lesion index and ciliostasis index. The remaining animals were observed for clinical symptoms associated with avian infectious bronchitis, including death, for 14 days after inoculation. The results are shown in Table 5 and Table 6.

**[TABLE 5]**

| Evaluation results of tissue lesion index and ciliostasis index 5 days after spray inoculation of IBV QX 1830029 HP70 strain and control vaccine in 1-day-old SPF chicks | | | | | | | |
|---|---|---|---|---|---|---|---|
| Group | Tissue lesion index^{A} (Mean±SD) | | | | Ciliostasis index^{B} (Mean±SD) | | |
| | Upper trachea | Middle trachea | Lower trachea | Kidney | Upper trachea | (Mean±SD) Middle trachea | Lower trachea |
| IBV QX 1830029 HP70 strain | 0.8±0.6 | 0.4±0.5 | 0.5±0.5 | 0.6±0.5 | 2.5±1.4 | 1.4±1.4 | 1.7±1.2 |
| Control vaccine group | 1.9±0.7 | 1.8±0.6 | 1.1±0.3 | 0.7±0.5 | 2.4±1.2 | 2.7±1.3 | 2.0±1.4 |
| Negative control group | 0.9±0.6 | 0.4±0.5 | 0.3±0.4 | 0.6±0.5 | 2.0±0.9 | 1.1±1.2 | 1.1±1.5 |

^{A} Histological lesion index of trachea and kidney collected 5 days after test vaccine inoculation (0, normal; 1, extensively focal; 2, multifocal; 3, diffuse)

^{B} Ciliostasis index of trachea collected 5 days after test vaccine inoculation (0, normal cilia; 1, ≤25% cilia damaged; 2, ≤50%; 3, ≤75%; 4, ≥75)

**[TABLE 6]**

| Observation results of clinical symptoms and death rate for 14 days after spray inoculation of IBV QX 1830029 HP70 strain and control vaccine in 1-day-old SPF chicks | | | |
|---|---|---|---|
| Group | Inoculation does | Clinical symptoms^{A} | Death^{B} |
| IBV QX 1830029 HP70 strain | 10^{4.5}EID₅₀/animal | 0/10 | 0/10 |
| Control vaccine group | 10 times the dose (x10 dose) | 0/10 | 0/10 |
| Negative control group | - | 0/10 | 0/10 |

^{A} Number of clinical symptoms expressed animals after spray inoculation /total number of tested animals

^{B} Number of dead animals after spray inoculation /total number of tested animals

As a result of evaluation on safety of the IBV QX 1830029 HP70 strain in spray inoculation, the present strain did not show clinical symptoms and deaths, like in the control vaccine group and the negative control group. Additionally, as a result of histological lesion comparison, the present strain showed a lower or similar degree of lesion compared with the control vaccine group and obtained similar results to the negative control group. It was therefore identified that the IBV QX 1830029 HP70 strain of the present invention is safe in spray inoculation in 1-day-old SPF chicks.

### 4-3. Evaluation on safety of IBV QX 1830029 HP70 strain in drinking water inoculation

To evaluate the safety of IBV QX 1830029 HP70 strain in drinking water inoculation, a tissue safety comparison test was conducted through drinking water inoculation of IBV QX 1830029 HP70 strain and a control vaccine. The control vaccine was selected from vaccines that are commercially available and widely used in Korea.

A total of 60 3-week-old SPF chickens were divided into an IBV QX 1830029 HP70 group, a control vaccine group, and a negative control group, each with 10 animals, and then underwent drinking water inoculation with 10^{4.5} EID₅₀/animal. The negative control group was inoculated with the same amount of sterilized phosphate buffered saline (PBS). At 5 day-post inoculation, 10 animals from each of the test and control groups were necropsied to collect the tracheas and kidneys, which were then fixed in 10% formalin, and thereafter, safety evaluation was conducted by determining the tissue lesion index and ciliostasis index. The results are shown in FIG. 7 and Table 8.

**[TABLE 7]**

| Observation results of clinical symptoms and death rate for 14 days after drinking water inoculation of IBV QX 1830029 HP70 strain and control vaccine in 3-week-old SPF chickens | | | |
|---|---|---|---|
| Group | Inoculation dose | Clinical symptoms^{A} | Death^{B} |
| IBV QX 1830029 HP70 | 10^{4.5}EID₅₀/animal | 0/10 | 0/10 |
| Control vaccine group | 10 times the dose (x10 dose) | 0/10 | 0/10 |
| Negative control group | PBS | 0/10 | 0/10 |

**[TABLE 8]**

| Evaluation results of tissue lesion index and ciliostasis index 5 days after drinking water inoculation of IBV QX 1830029 HP70 strain in 3-week-old SPF chickens | | | | | | | |
|---|---|---|---|---|---|---|---|
| Group | Tissue lesion index^{A} (Mean±SD) | | | | Ciliostsis^{B} (Mean±SD) | | |
| | Upper trachea | Middle trachea | Lower trachea | Kidney | Upper trachea | Middle trachea | Lower trachea |
| IBV QX 1830029 HP70 strain | 0.1±0.3 | 0.2±0.4 | 0.2±0.4 | 0.4±0.7 | 0.4±0.5 | 0.1±0.3 | 0.1±0.3 |
| Control vaccine group | 0.7±0.7 | 0.2±0.4 | 0.2±0.4 | 1.0±0.7 | 0.2±0.4 | 0.0±0.0 | 0.0±0.0 |
| Negative control group | 0.4±0.5 | 0.2+0.4 | 0.0±0.0 | 0.4±0.5 | 0.0±0.0 | 0.0±0.0 | 0.0±0.0 |

^{A} Histological lesion index of trachea and kidney collected 5 days after test vaccine inoculation (0, normal; 1, extensively focal; 2, multifocal; 3, diffuse)

^{B} Ciliostasis index of trachea collected 5 days after test vaccine inoculation (0, normal cilia; 1, ≤25% cilia damaged; 2, ≤50%; 3, ≤75%; 4, ≥75)

As a result of evaluation on safety of the IBV QX 1830029 HP70 strain in drinking water inoculation, the present strain did not show clinical symptoms and deaths, like in the control vaccine group and the negative control group. Additionally, as a result of histological lesion comparison, the present strain showed a lower or similar degree of lesion compared with the control vaccine group and obtained similar results to the negative control group. It was therefore identified that the IBV QX 1830029 HP70 strain is safe in drinking water inoculation in 3-week-old SPF chickens.

### 4-4. Test for minimum immunogenicity of IBV QX 1830029 HP70 strain

To determine the minimum immunogenicity of the IBV QX 1830029 HP70 strain, an evaluation test was conducted through eye drop inoculation. A total of 40 1-day-old SPF chicks were divided into 10 animals for each of groups: 10^{2.5} EID₅₀/animal inoculation group, 10^{3.0} EID₅₀/animal inoculation group, 10^{3.5} EID₅₀/animal inoculation group, and a negative control group, followed by inoculation. The negative control group was inoculated with the same amount of sterilized phosphate buffered saline (PBS). The clinical symptoms of each individual were observed for 21 days after inoculation, and at 21 day-post inoculation, all the animals of the test and control groups underwent blood collection to measure the IBV neutralization index, and the measurement results are shown in Table 9.

**[TABLE 9]**

| Observation results of IBV neutralization index and clinical symptoms in eye drop inoculation of IBV QX 1830029 HP70 strain in 1-day-old SPF chicks | | | |
|---|---|---|---|
| Classification | Inoculation dose | IBV neutralization index (Log10) ^{A} | Clinical symptoms^{B} |
| IBV QX 1830029 HP70 strain | 10^{2.5} EID₅₀/animal | 3.3 | 0/10 |
| | 10^{3.0} EID₅₀/animal | 4.0 | 0/10 |
| | 10^{3.5} EID₅₀/animal | 4.5 | 0/10 |
| Control group | - | - | 0/10 |

^{A} Neutralization index of each test vaccine against control group serum at 3 weeks after test vaccine inoculation

^{B} Number of animals showing clinical symptoms including death after test vaccine inoculation / Number of animals receiving vaccine.

As a result of the evaluation, the neutralization index exceeded 2.0 at all the doses in the eye drop inoculation of the IBV QX 1830029 HP70 strain, satisfying the Korean Standards of National Lot Release for Veterinary Biologics.

### Example 5: Evaluation on efficacy of IBV QX 1830029 HP70 strain in spray inoculation

To determine the efficacy of IBV QX 1830029 HP70 in 1-day-old chicks, an evaluation test was conducted. A total of 30 1-day-old SPF chicks were divided as shown in Table 10 and spray inoculated with 10^{3.5} EID₅₀/animal. At 21 day-post inoculation, all the animals of the vaccine inoculation group and the negative control underwent blood collection to determine the IBV neutralization index, and at 5 day-post challenge inoculation as shown in Table 10, the tracheas and kidneys were collected through autopsy to conduct virus re-isolation. The results are shown in Table 11.

**[TABLE 10]**

| Summary of spray efficacy test of IBV 1830029 HP70 strain in 1-day-old chicks | | | | |
|---|---|---|---|---|
| Classifi cation | Number of test animals | Age | Vaccine strain | Challenge strain |
| Group 1 | 10 | 1 day of age | IBV QX 1830029 HP70 | IBV QX 1830029 low passage |
| Group 2 | 9 | 1 day of age | - | IBV QX 1830029 low passage |
| Group 3 | 10 | 1 day of age | - | - |

**[TABLE 11]**

| Test results of IBV neutralization index and protective capacity against IBV QX 1830029 low passage in spray inoculation of IBV QX 1830029 HP70 strain in 1-day-old SPF chicks | | | | |
|---|---|---|---|---|
| Classification | IBV neutralization index^{A} | Clinical symptoms^{B} | Re-isolation rate^{c} | |
| | | | Trachea | Kidney |
| IBV QX 1830029 HP70 strain inoculation group | 5.2 | 1/10 | 1/10 | 1/10 |
| Control group | 0.0 | 9/9 | 9/9 | 9/9 |

^{A} Neutralization index of each test vaccine against control group serum at 3 weeks after test vaccine inoculation

^{B} Number of animals showing clinical symptoms including death after challenge inoculation / Number of animals receiving vaccine

^{C} Number of animals positive for challenge inoculation strain re-isolation / Number of animals receiving IBV challenge inoculation, in each organ

As a result of the evaluation, 1-day-old chicks spray inoculated with the IBV QX 1830029 HP70 strain at a dose of 10^{3.5} EID₅₀/animal showed a neutralization index exceeding 2.0, satisfying the Korean Standards of National Lot Release for Veterinary Biologics. The re-isolation test results confirmed a high protection rate. It was therefore identified that the spray inoculation of the IBV QX 1830029 HP70 strain was effective in 1-day-old SPF chicks.

### Example 6: Evaluation on efficacy of IBV QX 1830029 HP70 strain in drinking water inoculation

To determine the efficacy of IBV QX 1830029 HP70 in 3-week-old chickens, an evaluation test was conducted. A total of 30 3-week-old SPF chickens were divided as shown in Table 12 and underwent drinking water inoculation with 10^{3.5} EID₅₀/animal. At 21 day-post inoculation, all the animals of the vaccine inoculation group and the negative control underwent blood collection to determine the IBV neutralization index, and as shown in Table 12, underwent challenge inoculation with the IBV QX 1830029 low passage strain. At 5 day-post challenge inoculation, the tracheas and kidneys were collected through autopsy to conduct virus re-isolation. The results are shown in Table 13.

**[TABLE 12]**

| Summary of drinking water inoculation efficacy test of IBV 1830029 HP70 strain in 3-week-old chickens | | | | |
|---|---|---|---|---|
| Classifi cation | Number of tested animals | Age | Vaccine strain | Challenge strain |
| Group 1 | 10 | 3 weeks of age | IBV QX 1830029 HP70 | IBV QX 1830029 low passage |
| Group 2 | 10 | 3 weeks of age | - | IBV QX 1830029 low passage |
| Group 3 | 10 | 3 weeks of age | - | - |

**[TABLE 13]**

| Test results of IBV neutralization index and protective capacity against IBV QX 1830029 low passage in drinking water inoculation of IBV QX 1830029 HP70 strain in 3-week-old SPF chickens | | | | |
|---|---|---|---|---|
| Classification | IBV neutralization index^{A} | Clinical symptoms^{B} | Re-isola tion rate^{c} | |
| | | | Trachea | Kidney |
| IBV QX 1830029 HP70 strain inoculation group | 4.0 | 0/10 | 0/10 | 1/10 |
| Control group | - | 0/10 | 10/10 | 10/10 |

^{A} Neutralization index of each test vaccine against control group serum at 3 weeks after test vaccine inoculation

^{B} Number of animals showing clinical symptoms including death after challenge inoculation / Number of animals receiving vaccine

^{C} Number of animals positive for challenge inoculation strain re-isolation / Number of animals receiving IBV challenge inoculation, in each organ

As a result of the evaluation, 3-week-old chickens receiving drinking water inoculation with the IBV QX 1830029 HP70 strain at a dose of 10^{3.5} EID₅₀/animal showed a neutralization index exceeding 2.0, satisfying the Korean Standards of National Lot Release for Veterinary Biologics. The re-isolation test results confirmed a high protection rate. It was therefore identified that the drinking water inoculation of the IBV QX 1830029 HP70 strain was effective in 3-week-old chickens.

Although the present disclosure has been described in detail with reference to the specific features, it will be apparent to those skilled in the art that this description is only for a preferred embodiment and does not limit the scope of the present disclosure.

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Khav Co., Ltd
Khav Co., Ltd
120 Neungdong-ro,
Gwangjin-gu, Seoul

I. IDENTIFICATION OF THE MICROORGANISM
Identification reference given by the
DEPOSITOR: | Accession number given by the
INTERNATIONAL DEPOSITARY AUTHORITY:
IBV QX 1830029 HP70 strain | KCTC 15049BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 11, 2022
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):

KIM, Song-Gun, Director
Date: August 30, 2022

### [Accession Number]

Depository institution name: Korean Collection for Type Cultures (KCTC) at Korea Research Institute of Bioscience and Biotechnology
Accession No.: KCTC15049BP
Deposit date: 20220811

## Claims

1. An avian infectious bronchitis virus QX 1830029 HP70 strain deposited with accession number KCTC 15049 BP.

2. The strain of claim 1, wherein the QX 1830029 HP70 strain is attenuated from avian infectious bronchitis virus IBV/Korea/289/2018.

3. The strain of claim 1, wherein a spike protein of the QX 1830029 HP70 strain includes the amino acid sequence of SEQ ID NO: 6.

4. The strain of claim 1, wherein a spike protein of the QX 1830029 HP70 strain is encoded by the nucleotide sequence of SEQ ID NO: 4.

5. The strain of claim 1, wherein the avian infectious bronchitis virus QX 1830029 HP70 strain is obtained by attenuating IBV/Korea/289/2018 virus through sub-culturing.

6. The strain of claim 5, wherein the sub-culturing is conducted by heat treatment at a temperature of 60 to 70°C.

7. The strain of claim 5, wherein the sub-culturing is conducted in embryonated chicken eggs.

8. The strain of claim 5, wherein the sub-culturing is repeated for 50 to 70 passages.

9. A vaccine composition for preventing of avian infectious bronchitis virus, the composition comprising the avian infectious bronchitis virus QX 1830029 HP70 strain of any one of claims 1 to 8.

10. The vaccine composition of claim 5, wherein the vaccine is an attenuated vaccine.

11. The vaccine composition of claim 5, wherein the vaccine is administered by eye drop inoculation, spray inoculation, drinking water inoculation, or a combination thereof.
